# FASCICULE DE BREVET EUROPEEN

(11) **EP 1 308 130 B1**
(45) Date de publication et mention de la délivrance du brevet: **23.03.2011**
(21) Numéro de dépôt: 02292663.8
(22) Date de dépôt: 25.10.2002
(51) Int. Cl.: A61B 10/00, A61B 5/103

(54) **Dispositif de mesure et/ou d'analyse d'au moins un paramètre d'une portion externe du corps humain**
Vorrichtung zur Messung und/oder Analyse wenigstens eines Parameters von dem Aussenteil des menschlichen Körpers
Device for measuring and/or analysing at least one parameter of an external part of the human body

(30) Priorité: 06.11.2001 FR 0114334
(43) Date de publication de la demande: 07.05.2003
(73) Titulaire: L'Oréal, 75008 Paris (FR)
(72) Inventeur: Lefebvre, Marc André, 86600 Coulombiers (FR)
(74) Mandataire: Boulard, Denis

(56) Documents cités:
- WO-A-89/04630
- FR-A- 2 667 778
- US-A- 4 819 645

## Description

La présente invention a trait à un dispositif destiné à la mesure et/ou à l'analyse d'au moins un paramètre d'une portion externe du corps humain, notamment de la peau, des ongles ou des cheveux, en vue notamment de soumettre ladite portion à un traitement cosmétique.

Au sens de la présente invention, la portion externe du corps humain englobe également de la peau qui, en tout ou partie, a été reconstruite artificiellement, la mesure et/ou l'analyse pouvant se faire à tout moment lors du processus de reconstruction de la peau, notamment en vue d'en déterminer certaines caractéristiques physico-chimiques.

A titres exemples non limitatifs, un tel dispositif peut être utilisé pour connaître l'état du stratum corneum, analyser les sécrétions, excrétions, ou les odeurs, présentes à la surface de la peau ou des cheveux, ou analyser le contenu bioanalytique, bactériologique, ou enzymatique de la peau, des ongles, ou des cheveux.

Les éléments pouvant être prélevés par le dispositif selon l'invention peuvent être sous forme solide (cellules de peau morte, cuticules de cheveux, surface des ongles), liquide (sébum, eau) ou gazeuse (composés volatiles et/ou odorants).

Un tel dispositif peut être également utilisé pour l'évaluation de propriétés mécaniques de la peau, en particulier sa douceur, son élasticité ou son micro-relief, ce dernier pouvant être caractérisé notamment par le nombre et/ou la profondeur des rides ou micro-rides présentes à sa surface, ou par la densité et/ou la taille de ses pores.

Il est connu d'utiliser des éléments, notamment sous forme de "grattoirs", ou d'éléments adhésifs, destinés par exemple à prélever des cellules de peau morte à la surface de la peau. Bon nombre de tels dispositifs sont en plastique.

L'inconvénient de tels dispositifs tient au fait que certaines analyses, notamment celles impliquant des températures et/ou pressions importantes, ne peuvent être faites directement sur ces dispositifs, ces derniers n'étant en effet pas en mesure de résister à de telles températures.

De telles conditions de température se rencontrent notamment en chromatographie en phase gazeuse où la température à laquelle est soumise l'échantillon peut aller jusqu'à 450°C, voire 500°C. Des conditions de pressions importantes se rencontrent notamment en chromatographie en phase liquide où la pression peut aller jusqu'à 150 bars.

De ce fait, il est souvent nécessaire de transférer les éléments prélevés, notamment par aspiration, ou par tout autre moyen mécanique, physique ou chimique, sur un support adéquat ou dans un contenant approprié, apte à résister aux conditions, notamment de température et/ou de pression, auxquelles les éléments prélevés vont être exposés en vue de les qualifier et/ou de les quantifier.

Il en résulte des manipulations fastidieuses, susceptibles de causer la perte d'une partie des éléments prélevés. De ce fait, la mesure peut être imprécise.

En outre, cette incapacité de ces dispositifs connus à résister à des conditions extrêmes, que ce soit de température ou de pression, ou à des conditions liées à un environnement chimique agressif, rend difficile leur nettoyage dans des conditions telles, qu'ils puissent être réutilisés de façon fiable.

Enfin, et indépendamment de la capacité de ces dispositifs à résister à des conditions extrêmes, notamment de température et de pression, bon nombre de ces dispositifs connus comportent des impuretés, notamment des colles organiques, susceptibles de perturber la mesure.

La même problématique subsiste avec le dispositif décrit dans la demande de brevet FR-A-2 667 778, lequel dispositif permet de mettre en circulation un liquide sur la peau en vue de récupérer des éléments présents sur la surface de cette dernière. Le dispositif comprend en particulier des embouts destinés au raccordement du dispositif à une canalisation d'arrivée et à une canalisation de sortie du liquide. Le dispositif est de préférence réalisé en plastique. D'autres matériaux tels que la céramique sont évoqués comme matériaux pouvant être utilisés. Quoi qu'il en soit, un tel dispositif, lorsque les embouts sont collés, ne peut être soumis à des conditions de pression et de température, tels que décrits ci-avant, la colle en effet, n'étant pas capable de résister à de telles conditions.

D'autres éléments connus permettent de prélever des corps gras présents sur les cheveux ou à la surface de la peau, notamment le sébum. Ainsi, le brevet FR-A-2 368 708 suggère d'utiliser une plaque en verre dépoli pour collecter le sébum, en particulier à la surface du front d'un individu. L'inconvénient du verre sous sa forme massive tient au fait que, même dépoli, sa rugosité de surface est trop faible pour assurer un piégeage suffisant des éléments prélevés par la surface mise en contact avec la portion de peau à examiner. Ainsi, si l'on devait prélever des cellules de peau morte avec une plaque de verre, même dépoli, bon nombre de ces cellules tomberaient lors de son transport entre l'endroit ou le prélèvement est effectué et l'unité de traitement dans laquelle le dispositif est analysé. En outre, un passage accidentel d'un doigt sur la surface du verre provoquerait également l'enlèvement de bon nombre des éléments prélevés.

Le brevet US-A-5 433 214 décrit un dispositif destiné à quantifier le gras et/ou l'eau présent à la surface de la peau. Selon ce document, le dispositif est constitué d'une fine couche de dioxyde de silicium déposée par impression sur un substrat de couleur, notamment en papier ou PVC. Lorsque la couche de SiO₂ est au contact d'eau ou de gras, elle devient transparente, laissant apparaître la couleur du substrat.

Un tel dispositif, en raison notamment de la nature du substrat, et de tout autre élément organique qui le compose au moins en partie, ne peut être soumis à des analyses impliquant des conditions de température et/ou de pression importantes.

Il en va de même pour le dispositif décrit dans le brevet US-A-5 958 339.

Le document WO 89/04630 décrit un dispositif de prélèvement sous forme 'un élément solide qui, selon un mode de réalisation préféré, comprend un matériau de piégeage (pouvant être inorganique), imprégné sur un support pouvant être en tissu, ou en plastique perforé ou poreux, notamment du téflon. Encore une fois, un tel dispositif ne peut résister à des conditions de température et de pression tels qu'exemplifiés ci-avant.

Il en va de même pour le dispositif décrit dans le brevet US-A-4 819 645. Selon ce document, la structure faisant office de "piégeur" des substances à prélever est constituée d'un "liquid transfer medium" et d'un matériau réservoir dispersé dans ledit "liquid transfer medium". Ce dernier est sous forme d'un gel organique, notamment de l'Agarose ou du PVA. Il en résulte à nouveau une structure incapable de résister aux conditions de pression et de température évoquées ci-avant.

Le document US-A-6 283 978 décrit un système selon le préambule de la revendication 1.

Aussi, est-ce un des objets de l'invention que de réaliser un dispositif de mesure et/ou d'analyse qui permette de résoudre en tout ou partie les problèmes évoqués ci-avant en référence aux solutions conventionnelles.

C'est en particulier un objet de l'invention que de réaliser un tel dispositif qui puisse être utilisé à la fois pour le prélèvement de l'élément ou des éléments représentatifs du paramètre à évaluer, et pour l'analyse directe des éléments prélevés.

C'est un autre objet de l'invention que de réaliser un tel dispositif susceptible de résister à des conditions extrêmes, en particulier de température et de pression, telles que rencontrées notamment dans des dispositifs de chromatographie en phase gazeuse ou en phase liquide, ou telles qu'exigées pour en permettre un nettoyage satisfaisant.

C'est encore un autre objet que de réaliser un tel dispositif qui soit parfaitement nettoyable parfaitement en vue d'être réutilisé.

D'autres objets de l'invention apparaîtront de manière détaillée dans la description qui suit.

Ces objets sont atteints en réalisant un dispositif de mesure et/ou d'analyse d'au moins un paramètre, notamment biologique, mécanique, chimique ou physico-chimique d'une portion externe du corps humain, en particulier de la peau, des ongles ou des cheveux, tel que défini en revendication 1.

Par "poreuse", on entend apte à s'imprégner d'un réactif liquide ou d'un élément en phase liquide ou en phase gazeuse susceptible d'être présent à la surface de la portion externe à analyser.

Par "abrasive", on entend apte, par abrasion, à arracher des éléments solides présents à la surface de la portion externe à analyseur et de les retenir à l'intérieur des cavités formées entre les aspérités de sa surface de contact. De telles cavités englobent notamment des reliefs visibles à l'oeil nu.

Dans le cas d'une analyse par prélèvement, la surface peut être mise en contact, voire en appui sur la portion à analyser. C'est le cas notamment dans le cas de prélèvements d'éléments liquides ou solides. Pour des éléments en phase gazeuse, le contact n'est pas nécessaire, le prélèvement pouvant s'effectuer même quand la "surface de contact" ou "de prélèvement" est maintenue à faible distance de la portion à analyser.

Par "réalisé essentiellement en au moins un matériau inorganique" on entend une structure ne comportant pas de matériau autre qu'inorganique.

Bien entendu, le dispositif, notamment dans sa forme non modelable, peut être couplé de manière réversible à des moyens auxiliaires, tels que des moyens de préhension, des moyens d'entraînement, notamment rotatifs, des moyens de mesure ou des moyens de fixation qui eux, bien évidemment, peuvent être réalisés en des matériaux d'une nature différente.

Toutefois, le couplage avec de tels moyens auxiliaires doit être réversible de sorte que, le dispositif peut en être isolé, sans qu'il ne subsiste de parties ou résidus en un matériau autre qu'inorganique, et susceptibles notamment de fausser une mesure ou analyse effectuée sur le dispositif après application sur la portion mesure ou des moyens de fixation qui eux, bien évidemment, peuvent être réalisés en des matériaux d'une nature différente.

Toutefois, le couplage avec de tels moyens auxiliaires doit être réversible de sorte que, le dispositif peut en être isolé, sans qu'il ne subsiste de parties ou résidus en un matériau autre qu'inorganique, et susceptibles notamment de fausser une mesure ou analyse effectuée sur le dispositif après application sur la portion externe à analyser, ou de rendre le dispositif inadapté à des conditions extrêmes de température et/ou de pression, auxquelles il peut être soumis, par exemple en vue d'une analyse et/ou d'un nettoyage.

Ainsi, le dispositif peut être par exemple fixé par collage réversible à un support adhésif de manière à pouvoir être maintenu sur la peau pendant une plus longue période. Toutefois, la colle utilisée est telle, qu'elle adhère davantage au support qu'au dispositif de sorte que ce dernier puisse être séparé du support en vue d'une analyse nécessitant une résistance à des conditions de température et/ou de pression extrêmes, et ce, sans laisser de colle sur le dispositif.

La forme modelable du dispositif ne fait pas partie de l'invention.

De préférence, le dispositif, notamment sous sa forme non modelable, est d'épaisseur moyenne supérieure à 0,055 mm, et de préférence, supérieure à 0,1 mm, de préférence, supérieure à 0,5 mm, et de préférence encore, supérieure à 1 mm, et de préférence encore, supérieure à 2 mm.

Par "modelable" on entend, capable de se conformer intimement au profil de la portion externe à analyser, lorsqu'il est mis en appui sur ladite portion externe, et de conserver ledit profil lorsqu'il est séparé de ladite portion externe, de manière à former une empreinte de ladite portion externe. Le figeage du matériau peut se produire notamment à température ambiante.

De préférence, ledit dispositif, quand il n'est pas sous forme modelable, est obtenu par usinage, moulage, ou compactage.

De préférence, ladite surface comprend un matériau inorganique, autre qu'un métal lorsqu'il n'est pas sous forme fibreuse ou particulaire.

De préférence également, ledit dispositif est résistant à la chaleur et/ou à la pression.

Par "résistant à la chaleur" on entend, capable de résister à une température d'au moins 200° C, et de préférence d'au moins 400° C.

Par "résistant à la pression", on entend, capable de résister à une pression d'au moins 20 bars, et de préférence, d'au moins 50 bars, et de préférence encore, d'au moins 100 bars.

De préférence, ledit dispositif est configuré sous forme d'une structure à une seule couche.

De préférence, dans sa forme non modelable, le dispositif est formé d'une céramique, ou d'un élément en un matériau inorganique fibreux, notamment compacté, tel que de la laine de verre, ou de la laine de roche.

Par "céramique", on entend un élément en un matériau inorganique, non métallique et, notamment, obtenu en mettant en oeuvre lors de son processus d'élaboration, un traitement thermique.

Parmi les éléments solides pouvant être présents à la surface de la peau, des cheveux ou des ongles, peuvent être cités des cellules de peau morte, des cuticules, ou des pellicules.

Avantageusement, la rugosité de la surface de contact du dispositif correspond à celle d'une surface abrasive dont la granulométrie peut aller de P 12 à P 2500 telle que définie par les normes ISO 6344-1, ISO 6344-2 et ISO 6344-3.

Lors d'un prélèvement, en utilisant les propriétés abrasives de la surface du dispositif, ce dernier sera mis en appui sur la peau et entraîné en rotation ou en translation sur cette dernière.

La rugosité peut résulter de la structure même du matériau formant le dispositif, ou de la présence de reliefs adéquats présents sur la surface de prélèvement, notamment sous forme de losanges répartis de manière uniforme sur ladite surface. Ainsi, pour un élément sous forme d'une pastille de céramique de 2 mm d'épaisseur et de 15 mm de diamètre, la profondeur maximale des reliefs peut être de l'ordre de 1 mm.

A titre d'exemple, on a utilisé une pastille fournie par la société St. GOBAIN sous la référence AF997. En entraînant en rotation une telle structure, en appui sur la peau, il a été possible de récupérer entre 250 µg et 400 µg de stratum corneum. La vitesse de rotation de l'élément de prélèvement était comprise entre environ 50 et 70 Tr/mn.

Les éléments liquides ou volatils susceptibles d'être présents à la surface de la portion externe à analyser peuvent être des substances de nature lipophiles ou hydrophile. Il peut s'agir notamment de sébum ou d'eau.

Des essais satisfaisants ont été réalisés avec des structures sous forme de pastilles d'alumine ayant différentes porosités : 10%, 20% et 35%. De telles pastilles ont été fournies par la Société St GOBAIN sous les références 054999, 055000, 100360, et 100370.

Lors de ces essais, la pastille de céramique a été mise au contact du front pendant 10 mn. Ensuite, la pastille est plongée dans un solvant (dichlorométhane/méthanol). La quantité de sébum recueilli est d'environ 80 µg.

Après concentration, une partie aliquote du prélèvement est injectée dans un chromatographe, en vue de réaliser des dosages de cholestérol et de Squalene.

La mesure ou l'analyse peut passer par l'enregistrement d'au moins une grandeur, d'une image, d'une force, ou de courants, obtenus en réponse à la mise en contact, statique ou dynamique, du dispositif de mesure avec la portion externe à analyser.

Alternativement, la mesure ou l'analyse passe par le prélèvement d'éléments, sous forme liquide, solide ou gazeuse, et présents à la surface de la peau, tels que des phases volatiles et/ou odorantes, des sécrétions ou excrétions produites par les glandes sébacées ou sudorales, ou des cellules de peau morte.

Alternativement encore, la mesure ou l'analyse passe par un changement d'état, notamment de couleur, de réactifs contenus dans le dispositif, lorsque ce dernier est mis au contact, ou du moins en regard, de la portion à analyser.

Avec le dispositif selon l'invention, il est donc possible d'avoir des informations sur des paramètres mécaniques, biochimiques, bactériologiques, enzymatiques et/ou analytiques de la portion externe à analyser. Il est possible également de réaliser des bilans correspondants.

Le dispositif, pendant sa mise en contact avec la portion à examiner, ou après avoir été mis en contact statique et/ou dynamique avec la portion externe à examiner, peut faire l'objet de différentes analyses.

Il peut s'agir en premier lieu d'une simple observation du dispositif, laquelle permet de voir ce qui a été prélevé à la surface de la peau, ou d'observer un changement d'état, notamment de couleur, d'un réactif contenu dans le dispositif.

Une telle observation peut se faire par tout moyen optique ou microscopique. L'analyse peut être faite notamment à l'oeil nu, au moyen d'un microscope ou au moyen d'un analyseur d'images.

Ainsi, par exemple, peut être évalué le niveau d'acidité de la peau en observant la couleur du dispositif, résultant du changement de couleur d'un réactif contenu dans le dispositif, ou le degré de sécheresse de la peau en dénombrant les cellules de peau morte prélevées et/ou en évaluant leur taille.

De même, après mise en contact ou en regard du dispositif d'analyse avec la peau ou les cheveux pendant une durée suffisante, le dispositif d'analyse selon l'invention peut être soumis à des moyens d'observation spectrophotométriques, UV, Fluorescence, Infrarouge, ou Raman. De tels moyens d'observation sont particulièrement adaptés à l'analyse de morceaux de stratum corneum prélevés par le dispositif d'analyse.

Alternativement, il peut être procédé à une analyse en direct, notamment au moyen d'un chromatographe en phase gazeuse ou en phase liquide (normale ou inverse). On peut également soumettre le dispositif à un examen aux rayons X pour analyse élémentaire. De tels examens permettent notamment d'obtenir des cartographies des paramètres analytiques du stratum corneum.

Le dispositif peut être également utilisé pour collecter des bactéries présentes sur la peau, en vue de déterminer "l'équipement" bactérien d'un individu. Après application du dispositif sur la peau pendant une période prédéterminée, on procède à un ensemencement sur un milieu de culture.

La même chose peut être faite pour les enzymes. Pour ce faire, on fait tourner par exemple une face rugueuse de la céramique en appui sur la peau de manière à récupérer un peu de stratum corneum, lequel fera l'objet ensuite de ladite analyse enzymatique.

En outre, comme on le verra par la suite, le dispositif peut être couplé à une instrumentation, notamment à un capteur de force, destiné à mesurer la force nécessaire pour entraîner le dispositif selon un mouvement prédéterminé, lorsqu'il est en contact, voire en appui, sur la peau. On peut ainsi mesurer des propriétés mécaniques de la peau, telles que sa douceur.

Alternativement, une telle instrumentation peut compter des électrodes capables de mesurer l'évolution de certains paramètres tels que le pH ou un signal électrochimique.

Alternativement encore, une telle instrumentation peut comporter des électrodes, couplées par exemple à des biocapteurs enzymatiques présents dans le dispositif, de manière à enregistrer des signaux, en vue par exemple d'enregistrer des variations de paramètres particuliers.

Bien entendu, toute cette instrumentation, lorsqu'elle est intégrée au dispositif, en particulier dans sa forme non modelable, est réalisée également en matériau inorganique, notamment en certains métaux, capables de résister à des conditions de température et/ou de pression élevées.

De préférence, à l'exception de ces capteurs ou autres moyens accessoires intégrés éventuellement dans le dispositif, ce dernier est réalisé en un matériau inorganique non métallique.

La forme du dispositif est choisie en fonction de la nature de l'analyse à réaliser, et de la surface à examiner. Il peut être notamment réalisé sous forme d'un bloc, d'un disque, d'une ou plusieurs lames, ou d'une feuille. Typiquement, son épaisseur peut être comprise entre 0,055 mm et 20 mm, et de préférence, entre 0,5 mm et 10 mm.

En fonction notamment du profil de la surface sur laquelle le dispositif est destiné à être appliqué, on configurera ce dernier sous forme flexible ou non.

Avantageusement, le dispositif est configuré sous forme d'un disque dont le diamètre va de 0,5 mm à 50 mm, et de préférence, de 5 mm à 30 mm.

Comme mentionné précédemment, le dispositif peut contenir un ou plusieurs réactifs aptes à réagir avec au moins un élément présent à la surface de la portion externe à analyser, ou au voisinage de cette dernière.

A titres d'exemples, ledit réactif est sensible au pH, à l'urée, à l'ammoniaque, à l'albumine, au sucre, aux lipides, ou à l'acide lactique.

Le dispositif d'analyse selon l'invention peut être jetable ou non. Dans ce dernier cas, il pourra, en vue d'une nouvelle utilisation, être nettoyé au moyen d'un traitement thermique et/ou chimique adéquat.

Selon un mode de réalisation particulier, le dispositif comprend au moins un capteur destiné notamment à mettre en évidence au moins un paramètre, notamment un paramètre biochimique, ledit capteur étant couplé ou étant apte à être couplé notamment à un intégrateur et à un enregistreur.

A titre d'exemples de capteurs, peuvent être cités les biocapteurs aptes à mettre en évidence des paramètres biochimiques tels que la protéase, la phosphatase, la catalase, l'estérase, ou la glucosidase, etc..

Selon un mode de réalisation avantageux, le dispositif selon l'invention est constitué d'une céramique usinable.

A titre d'exemples, on utilise une céramique à base de graphite, de silice, d'alumine, de dioxyde de titane, de trioxyde de fer, d'oxyde de calcium, de dioxyde de potassium, d'oxyde de sodium, ou d'un mélange de tels matériaux.

Ainsi, à la manière d'une pâte à modeler, la matériau céramique peut être appliqué sur la peau et conformé au profil de celle-ci. Généralement, un tel matériau se fige à la température ambiante. Après figeage, le dispositif est retiré. On observe sur la surface qui a été mise en contact avec la peau, une image du micro-relief de cette dernière, et en particulier des rides, micro-rides, ou des pores présents à sa surface. L'image ainsi transférée peut alors faire l'objet d'une observation optique, ou d'une analyse d'image.

Dans sa version modelable, le dispositif peut faire, en outre ou alternativement, l'objet d'analyses du type de celles décrites précédemment, et impliquant des conditions de température et/ou de pression extrêmes.

Une telle céramique moulable peut être à base de Silice fondue, d'Oxyde de Zirconium, de Carbure de Silicium, d'Alumine, ou d'un mélange de tels matériaux.

Selon un autre aspect de l'invention on réalise un procédé pour la prescription d'un traitement cosmétique à appliquer sur une portion externe du corps humain, notamment la peau, les ongles, ou les cheveux, ledit procédé consistant à prescrire au moins un produit cosmétique à appliquer sur ladite portion externe, en fonction du résultat d'une analyse et/ou d'une mesure d'un paramètre de ladite portion externe obtenu à partir d'un dispositif d'analyse et/ou de mesure selon l'invention.

Par "produit cosmétique", on entend toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).

A titre d'exemples non limitatifs de produits cosmétiques, on peut citer tout produit destiné à réduire les signes du vieillissement de la peau ou du cheveu, notamment les rides, à hydrater la peau, à nettoyer et/ou à nourrir et/ou à entretenir la peau ou les cheveux, à désodoriser la peau, à la préparer à une exposition solaire, à en renforcer l'élasticité, à en améliorer la douceur, etc..

Par "traitement cosmétique" on entend tout traitement au moyen d'un produit cosmétique tel que défini ci-avant.

Selon encore un autre aspect de l'invention, on réalise un procédé de traitement cosmétique d'une portion externe du corps humain, notamment la peau, les ongles, ou les cheveux, comprenant les étapes suivantes :
a) mesurer et/ou analyser au moins un paramètre de ladite portion externe au moyen d'un dispositif d'analyse et/ou de mesure selon l'invention ; et
b) en fonction du résultat de la mesure et/ou de l'analyse faite en a), appliquer sur ladite portion externe au moins un produit cosmétique supposé avoir une action bénéfique sur ledit paramètre.

Selon encore un autre aspect de l'invention, on réalise un procédé de détermination de l'efficacité d'un traitement cosmétique appliqué sur une portion externe du corps humain, notamment la peau, les ongles, ou les cheveux, comprenant les étapes suivantes :
a) mesurer et/ou analyser au moins un paramètre de ladite portion externe au moyen d'un dispositif d'analyse et/ou de mesure selon l'invention ;
b) en fonction du résultat de la mesure et/ou de l'analyse faite en a), procéder à au moins une application sur ladite portion externe d'au moins un produit cosmétique supposé avoir une action bénéfique sur ledit paramètre ; et
c) mesurer et/ou analyser à nouveau ledit paramètre au moyen dudit dispositif.

Si à l'issue de l'étape c), il ressort que les résultats sont inexistants ou insuffisants, un traitement cosmétique différent peut être prescrit, en complément ou en remplacement du premier.

L'application du dispositif d'analyse et/ou de mesure sur la zone à examiner peut être effectuée directement par l'individu à tester, soit chez lui, soit sur un point de vente, soit dans un centre spécialisé, notamment chez une esthéticienne.

Notamment lorsque la "lecture" du dispositif se fait par une analyse visuelle, en particulier en vue de détecter un simple changement de couleur, cette "lecture" peut être faite directement par ledit individu. Une telle lecture peut être facilitée en fournissant avec le dispositif, une échelle colorée.

L'individu peut alors communiquer le résultat observé, par tout moyen, à un professionnel, soit présent à l'endroit où est faite l'analyse, soit à distance. Le professionnel, établira un diagnostic, et, le cas échéant, prescrira un traitement cosmétique censé avoir une action sur le paramètre ayant fait l'objet de l'analyse.

La "lecture" du dispositif peut être aussi faite par un professionnel présent sur le point de vente ou dans le centre spécialisé.

Alternativement, lorsque l'équipement requis pour procéder à l'analyse du dispositif est plus lourd, l'individu peut mettre le dispositif dans une enveloppe et l'envoyer à un centre de traitement délocalisé où sera faite la "lecture" du dispositif, et où sera établi un diagnostic.

Alternativement encore, l'individu peut scanner une image collectée par le dispositif, notamment une image du relief de la peau, et l'envoyer, notamment via INTERNET, à un centre délocalisé, lequel établira un diagnostic et prescrira le cas échéant un traitement cosmétique.

L'invention consiste, mises à part les dispositions exposées ci-dessus, en un certain nombre d'autres dispositions qui seront explicitées ci-après, à propos d'exemples de réalisation non limitatifs, décrits en référence aux figures annexées, parmi lesquelles :
- la figure 1 représente une vue en perspective d'un dispositif d'analyse selon un premier mode de réalisation de l'invention ;
- la figure 2 représente une vue en perspective d'un dispositif d'analyse selon un second mode de réalisation de l'invention ;
- la figure 3 représente une vue en perspective d'un dispositif d'analyse selon un troisième mode de réalisation qui ne fait pas partie de l'invention;
- la figure 4 illustre un mode d'utilisation d'un dispositif de mesure selon l'invention ; et
- la figure 5 illustre un autre mode d'utilisation d'un dispositif de mesure selon l'invention.

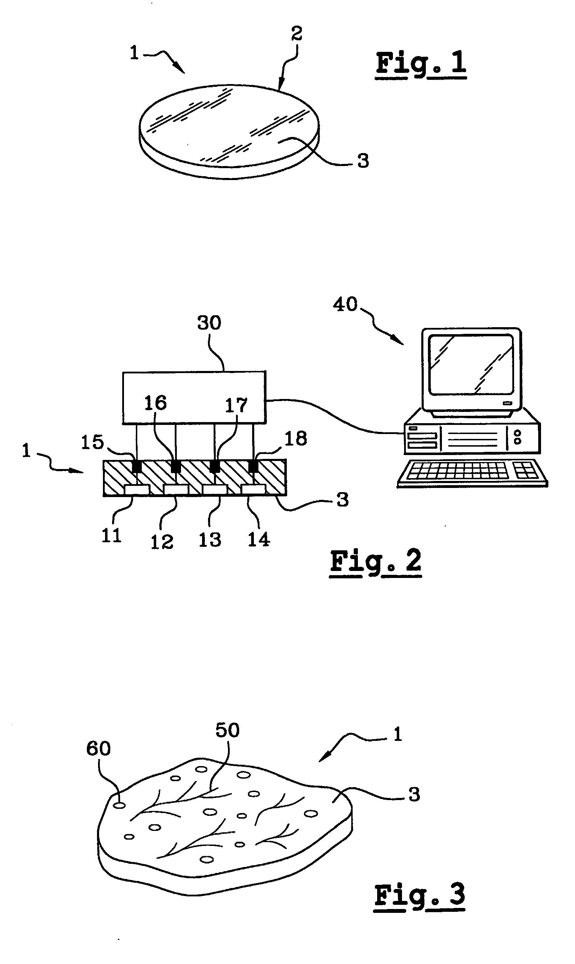

Le dispositif d'analyse 1 représenté à la figure 1 est configuré sous forme d'un disque 2 d'environ 1 cm de diamètre, et d'environ 4 mm d'épaisseur. Le disque est réalisé en une céramique usinée, commercialisée sous la référence commerciale 902 par la Société COTRONICS®. Une telle céramique est formée d'un mélange de silice, d'alumine, de dioxyde de titane, de dioxyde de fer, d'oxyde de calcium, de dioxyde de potassium, et d'oxyde de sodium.

La surface 3 du disque 2, destinée à être mise au contact de la peau, est rugueuse de sorte que, en la mettant en appui sur la surface de la peau, et en la déplaçant relativement à cette dernière, des cellules de peau morte soient arrachées à la peau et piégées par la surface rugueuse 3.

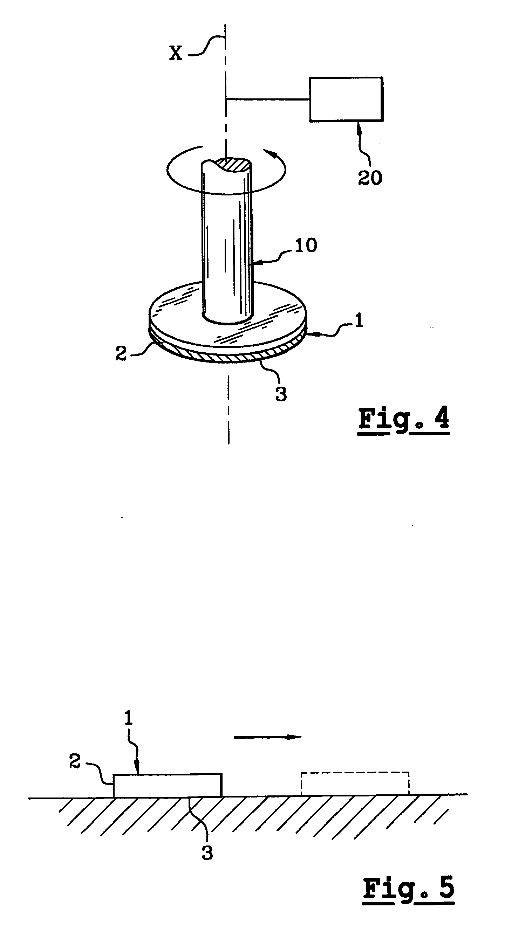

Le déplacement de la surface 3 relativement à la peau peut se faire de la manière illustrée à la figure 4, c'est à dire en rotation autour d'un axe X perpendiculaire au plan de ladite surface 3.

A cet effet, le dispositif de mesure 1 peut être monté par collage non permanent, ou par tout autre moyen réversible, sur un organe rotatif 10 couplé à un moteur 20.

Alternativement, le déplacement de la surface 3 du disque 2 sur la surface de la peau peut se faire de la manière représentée à la figure 5, c'est à dire selon un mouvement de translation.

Dans le mode de réalisation de la figure 2, le dispositif de mesure en céramique 1 comprend, au voisinage de la surface 3, destinée à être mise au contact de la peau, une pluralité de biocapteurs 11-14 aptes chacun à mettre en évidence un paramètre biochimique particulier. Ainsi, le biocapteur 11 est sensible à la protéase. Le biocapteur 12 est sensible à la phosphatase. Le biocapteur 13 est sensible à la catalase. Le biocapteur 14 sensible à la glucosidase.

Chacun des capteurs 11-14 est relié à une électrode respective 15-18 destinée à générer des signaux électrochimiques correspondants. Les signaux générés par les électrodes 15-18 sont collectés et convertis en signaux numériques par un enregistreur/décodeur 30, relié à un ordinateur 40. Un tel mode de réalisation permet de "monitorer" les variations dans le temps des paramètres analysés.

Dans le mode de réalisation de la figure 3, le dispositif 1 est constitué d'un morceau de céramique modelable, que l'on applique sur la peau, et que l'on conforme au profil de cette dernière. La céramique se fige à température ambiante. Après enlèvement, la surface 3 qui a été au contact de la peau comporte l'image des rides ou ridules 50 et des pores 60 présents à la surface de la peau. La lecture du dispositif se fait, soit visuellement, soit par analyse d'image.

Dans cet exemple de la figure 3, le matériau formant le dispositif d'analyse 1 est une céramique moulable commercialisée sous la référence RESCORE® par la société COTRONICS®.

Dans la description détaillée qui précède, il a été fait référence à des modes de réalisation préférés de l'invention. Il est évident que des variantes peuvent y être apportées sans s'écarter de l'étendue de l'invention telle que revendiquée ci-après.

## Revendications

1. Dispositif (1) pour l'analyse et/ou la mesure d'au moins un paramètre, notamment biologique, mécanique, chimique ou physico-chimique, d'une portion externe du corps, notamment de la peau, des ongles, ou des cheveux, le dispositif (1) comprenant au moins une surface (3) destinée à être mise en regard ou au contact de ladite portion externe, ledit dispositif étant non modelable sur ladite portion externe, et ne comportant pas de matériau autre qu'inorganique, **caractérisé en ce que** ladite surface (3) est abrasive et/ou poreuse et comprend un matériau inorganique, autre que le verre lorsqu'il n'est pas sous forme fibreuse ou particulaire, ledit dispositif étant formé d'une céramique à base de graphite, de silice, d'alumine, de dioxyde de titane, de trioxyde de fer, d'oxyde de calcium, de dioxyde de potassium, d'oxyde de sodium, ou d'un mélange de tels matériaux, ou d'un élément en un matériau inorganique fibreux compacté, tel que de la laine de verre, ou de la laine de roche.

2. Dispositif selon la revendication 1 **caractérisé en ce que** la surface (3) comprend un matériau inorganique autre qu'un métal lorsqu'il n'est pas sous forme fibreuse ou particulaire.

3. Dispositif (1) selon la revendication 1 ou 2 **caractérisé en ce que** le dispositif est d'épaisseur moyenne supérieure à 0,055 mm.

4. Dispositif selon l'une quelconque des revendications 1 à 3 **caractérisé en ce que** le dispositif est capable de résister à une chaleur d'au moins 200° C et/ou à une pression d'au moins 20 bars.

5. Dispositif (1) selon l'une quelconque des revendications 1 à 4 **caractérisé en ce qu'**il est réalisé sous forme d'un élément dont l'épaisseur est comprise entre 0,055 mm et 20 mm, et de préférence, entre 0,5 mm et 10 mm.

6. Dispositif (1) selon l'une quelconque des revendications 1 à 5 **caractérisé en ce qu'**il est configuré sous forme d'un disque (2) dont le diamètre va de 0,5 mm à 50 mm, et de préférence, de 5 mm à 30 mm.

7. Dispositif (1) selon la revendication 1 **caractérisé en ce que** ladite surface (3) est poreuse et contient au moins un réactif apte à réagir avec au moins un élément présent à la surface de ladite portion externe ou au voisinage de cette dernière.

8. Dispositif (1) selon la revendication 7 **caractérisé en ce que** ledit réactif est sensible au pH, à l'urée, à l'ammoniaque, à l'albumine, au sucre, aux lipides, ou à l'acide lactique.

9. Dispositif (1) selon l'une quelconque des revendications précédentes **caractérisé en ce que** ladite surface est abrasive, la rugosité de ladite surface (3) correspondant à celle d'une surface abrasive dont la granulométrie est comprise entre P 12 et P 2500.

10. Dispositif (1) selon l'une quelconque des revendications 1 à 9 **caractérisé en ce qu'**il comprend au moins un capteur (11-14) destiné notamment à mettre en évidence au moins un paramètre, notamment un paramètre biochimique, de ladite portion externe.

11. Procédé de traitement cosmétique d'une portion externe du corps humain, notamment la peau, les ongles, ou les cheveux, comprenant les étapes suivantes :
a) mesurer et/ou analyser au moins un paramètre de ladite portion externe au moyen d'un dispositif d'analyse et/ou de mesure (1) selon l'une quelconque des revendications 1 à 10 ; et
b) en fonction du résultat de la mesure et/ou de l'analyse faite en a) appliquer sur ladite portion externe au moins un produit cosmétique supposé avoir une action bénéfique sur ledit paramètre.

12. Procédé pour la détermination de l'efficacité d'un traitement cosmétique appliqué sur une portion externe du corps humain, notamment la peau, les ongles ou les cheveux, comprenant les étapes suivantes :
a) mesurer et/ou analyser au moins un paramètre de ladite portion externe au moyen d'un dispositif d'analyse et/ou de mesure (1) selon l'une quelconque des revendications 1 à 10;
b) en fonction du résultat de l'analyse et/ou de la mesure faite en a), procéder à au moins une application sur ladite portion externe d'au moins un produit cosmétique supposé avoir une action bénéfique sur ledit paramètre ; et
c) mesurer et/ou analyser à nouveau ledit paramètre au moyen dudit dispositif d'analyse (1).

## Claims

1. Device (1) for measuring and/or analyzing at least one parameter, in particular biological, mechanical, chemical or physicochemical parameter, of an external portion of the body, in particular of the skin, the nails or the hair, the device (1) comprising at least one surface (3) intended to be placed opposite or brought into contact with said external portion, said device being unable to be modeled to said external portion, and not comprising any material other than inorganic material, **characterized in that** said surface (3) is abrasive and/or porous and comprises an inorganic material other than glass when it is not in a fibrous or particulate form, said device being made of a ceramic based on graphite, on silica, on alumina, on titanium dioxide, on iron trioxide, on calcium oxide, on potassium dioxide, on sodium oxide or on a mixture of such materials, or of an element made of a fibrous, compacted, inorganic material, such as glass wool or rockwool.

2. Device according to Claim 1, **characterized in that** the surface (3) comprises an inorganic material other than a metal when it is not in a fibrous or particulate form.

3. Device (1) according to Claim 1 or 2, **characterized in that** the device has an average thickness of greater than 0.055 mm.

4. Device according to any one of Claims 1 to 3, **characterized in that** the device is capable of withstanding a heat of at least 200°C and/or a pressure of at least 20 bar.

5. Device (1) according to any one of Claims 1 to 4, **characterized in that** it is made in the form of an element which has a thickness of between 0.055 mm and 20 mm, and preferably of between 0.5 mm and 10 mm.

6. Device (1) according to any one of Claims 1 to 5, **characterized in that** it is configured in the form of a disk (2) with a diameter ranging from 0.5 mm to 50 mm, and preferably from 5 mm to 30 mm.

7. Device (1) according to Claim 1, **characterized in that** said surface (3) is porous and contains at least one reagent capable of reacting with at least one element present at the surface of said external portion, or in the proximity of the latter.

8. Device (1) according to Claim 7, **characterized in that** said reagent is sensitive to pH, to urea, to aqueous ammonia, to albumin, to sugar, to lipids or to lactic acid.

9. Device (1) according to any one of the preceding claims, **characterized in that** said surface is abrasive, the roughness of said surface (3) corresponding to that of an abrasive surface the grain size of which is between P 12 and P 2500.

10. Device (1) according to any one of Claims 1 to 9, **characterized in that** it comprises at least one sensor (11-14) intended in particular to demonstrate at least one parameter, in particular a biochemical parameter, of said external portion.

11. Method of cosmetic treatment of an external portion of the human body, in particular the skin, the nails or the hair, comprising the following steps:
a) measuring and/or analyzing at least one parameter of said external portion by means of an analytical and/or measuring device (1) according to any one of Claims 1 to 10; and
b) depending on the result of the measurement and/or of the analysis carried out in a), applying at least one cosmetic product supposed to have a beneficial action on said parameter to said external portion.

12. Method for determining the effectiveness of a cosmetic treatment applied to an external portion of the human body, in particular the skin, the nails or the hair, comprising the following steps:
a) measuring and/or analyzing at least one parameter of said external portion by means of an analytical and/or measuring device (1) according to any one of Claims 1 to 10;
b) depending on the result of the analysis and/or of the measurement carried out in a), performing at least one application of at least one cosmetic product supposed to have a beneficial action on said parameter to said external portion; and
c) measuring and/or analyzing said parameter again by means of said analytical device (1).

## Patentansprüche

1. Vorrichtung (1) für die Analyse und/oder die Messung mindestens eines Parameters, insbesondere biologischen, mechanischen, chemischen oder physikalisch-chemischen Parameters, eines äußeren Teils des Körpers, insbesondere der Haut, der Nägel oder der Haare, wobei die Vorrichtung (1) mindestens eine Oberfläche (3) umfasst, die dafür vorgesehen ist, diesem äußeren Teil gegenüber angeordnet oder mit diesem äußeren Teil in Kontakt gebracht zu werden, wobei die Vorrichtung auf dem äußeren Teil nicht anpassbar ist und kein anderes Material als ein anorganisches Material umfasst, **dadurch gekennzeichnet, dass** die Oberfläche (3) abrasiv und/oder porös ist und ein anderes anorganisches Material als das Glas umfasst, wenn es nicht faserförmig oder partikelförmig vorliegt, wobei die Vorrichtung aus einer Keramik auf der Basis von Graphit, Kieselsäure, Aluminiumoxid, Titandioxid, Eisentrioxid, Calciumoxid, Kaliumdioxid, Natriumoxid oder einem Gemisch derartiger Materialien oder aus einem Element aus einem verdichteten faserigen anorganischen Material, wie Glaswolle oder Steinwolle, gebildet ist.

2. Vorrichtung nach Anspruch 1, **dadurch gekennzeichnet, dass** die Oberfläche (3) ein anders anorganisches Material als ein Metall umfasst, wenn es nicht faserförmig oder partikelförmig ist.

3. Vorrichtung nach Anspruch 1 oder 2, **dadurch gekennzeichnet, dass** die Vorrichtung eine mittlere Dicke größer als 0,055 mm aufweist.

4. Vorrichtung nach einem der Ansprüche 1 bis 3, **dadurch gekennzeichnet, dass** die Vorrichtung dazu imstande ist, eine Hitze von mindestens 200 °C und/oder einen Druck von mindestens 20 bar auszuhalten.

5. Vorrichtung (1) nach einem der Ansprüche 1 bis 4, **dadurch gekennzeichnet, dass** sie in Form eines Elements hergestellt ist, dessen Dicke im Bereich von 0,055 mm bis 20 mm und vorzugsweise im Bereich von 0,5 mm bis 10 mm liegt.

6. Vorrichtung (1) nach einem der Ansprüche 1 bis 5, **dadurch gekennzeichnet, dass** sie in Form einer Scheibe (2) gestaltet ist, deren Durchmesser im Bereich von 0,5 mm bis 50 mm und vorzugsweise 5 mm bis 30 mm liegt.

7. Vorrichtung (1) nach Anspruch 1, **dadurch gekennzeichnet, dass** die Oberfläche (3) porös ist und mindestens ein Reagens enthält, das imstande ist, mit mindestens einem Bestandteil zu reagieren, der auf der Oberfläche dieses äußeren Teils oder in der Nähe von letzterem vorhanden ist.

8. Vorrichtung (1) nach Anspruch 7, **dadurch gekennzeichnet, dass** das Reagens empfindlich gegenüber dem pH-Wert, Harnstoff, Ammoniak, Albumin, Zucker, Lipiden oder Milchsäure ist.

9. Vorrichtung (1) nach einem der vorhergehenden Ansprüche, **dadurch gekennzeichnet, dass** die Oberfläche abrasiv ist, wobei die Rauheit der Oberfläche (3) der Rauheit einer abrasiven Oberfläche entspricht, deren Korngröße im Bereich von P 12 bis P 2500 liegt.

10. Vorrichtung (1) nach einem der Ansprüche 1 bis 9, **dadurch gekennzeichnet, dass** sie mindestens einen Sensor (11-14) umfasst, der insbesondere dafür vorgesehen ist, mindestens einen Parameter, insbesondere einen biochemischen Parameter, dieses äußeren Bereichs, nachzuweisen.

11. Verfahren zur kosmetischen Behandlung eines äußeren Teils des menschlichen Körpers, insbesondere der Haut, der Nägel oder der Haare, das die folgenden Schritte umfasst:
a) Messen und/oder Analysieren mindestens eines Parameters dieses äußeren Teils mit Hilfe einer Vorrichtung für die Analyse und/oder Messung (1) nach einem der Ansprüche 1 bis 10; und
b) in Abhängigkeit vom Ergebnis der in a) durchgeführten Messung und/oder Analyse Aufbringen mindestens eines kosmetischen Produkts, von dem angenommen wird, dass es eine vorteilhafte Wirkung auf den Parameter hat, auf den äußeren Teil.

12. Verfahren für die Ermittlung der Wirksamkeit einer kosmetischen Behandlung, die auf einem äußeren Teil des menschlichen Körpers, insbesondere der Haut, den Nägeln oder den Haaren, durchgeführt wird, das die folgenden Schritte umfasst:
a) Messen und/oder Analysieren mindestens eines Parameters dieses äußeren Teils mit Hilfe einer Vorrichtung für die Analyse und/oder Messung (1) nach einem der Ansprüche 1 bis 10; und
b) in Abhängigkeit vom Ergebnis der in a) durchgeführten Messung und/ oder Analyse Fortfahren mit dem mindestens einmaligen Auftragen mindestens eines kosmetischen Produkts, von dem angenommen wird, dass es eine vorteilhafte Wirkung auf den Parameter hat, auf den äußeren Teil; und
c) erneutes Messen und/oder Analysieren des Parameters mit Hilfe der Analysevorrichtung (1).
